# FASCICULE DE BREVET EUROPEEN

(11) **EP 0 432 251 B1**
(45) Date de publication et mention de la délivrance du brevet: **23.08.1995**
(21) Numéro de dépôt: 90910141.2
(22) Date de dépôt: 20.06.1990
(51) Int. Cl.: A61B 5/12

(54) **APPAREIL DESTINE A LA MESURE DE LA SENSIBILITE D'UN SUJET A LA PERCEPTION D'UNE VIBRATION**
VORRICHTUNG ZUM MESSEN DER FÜHLUNGSEMPFINDLICHKEIT EINER VERSUCHSPERSON
APPARATUS FOR MEASURING THE SENSITIVITY OF A SUBJECT'S PERCEPTION OF VIBRATION

(30) Priorité: 23.06.1989 FR 8908397
(43) Date de publication de la demande: 19.06.1991
(73) Titulaire: I.N.R.S. - INST. NAT. DE RECHERCHE ET DE SECURITE POUR LA PREVENTION DES ACCIDENTS DU TRAVAIL ET DES MALADIES PROFESSIONNELLES, 75680 Paris Cédex 14 (FR)
(72) Inventeur: MEYER-BISCH, Christian, F-54000 Nancy (FR)
(74) Mandataire: Derambure, Christian
(86) Numéro de dépôt international: FR9000450
(87) Numéro de publication internationale: WO9100055

(56) Documents cités:
- GB-A- 2 033 641
- US-A- 3 809 811
- US-A- 4 038 496
- US-A- 4 615 007
- MEDICAL AND BIOLOGICAL ENGINEERING. vol. 11, no. 5, septembre 1973, STEVENAGEGB pages 603 - 608; H.IDE et al.: "Relation between vibratory sensibility andelectric signal of living body" voir le document en entier

## Description

L'invention concerne un appareil destiné à la mesure de la sensibilité d'un sujet à la perception d'une vibration.

Si la vibration engendre un son, un tel appareil peut être utilisé pour mesurer l'acuité auditive d'un sujet et il s'agit alors d'un audiomètre. Les audiomètres les plus répandus sont ceux destinés à l'audiométrie tonale en conduction aérienne qui consiste à appliquer des sons purs à des niveaux de pression acoustique variables au moyen d'écouteurs de manière à déterminer le seuil de perception auditive du sujet pour différentes fréquences.

En tant qu'audiomètre, l'appareil de l'invention peut également être utilisé pour réaliser des mesures audiométriques par conduction osseuse.

Il peut encore être utilisé pour réaliser des mesures de seuil de sensibilité périphérique.

La méthode audiométrique manuelle est encore à ce jour la plus utilisée. Elle met en oeuvre un générateur de sons qui émet, pour une série de fréquences données, des sons de niveaux variables. Par exemple, en actionnant une presselle, le sujet signale à l'examinateur le franchissement du seuil d'audition. Il est ainsi possible de tracer l'audiogramme correspondant. La précision de cet audiogramme dépend du nombre de fréquences utilisées (en général sept ou neuf), du pas de progression du niveau, de la coopération du sujet examiné, du niveau de bruit ambiant, de l'examinateur qui doit être bien entraîné et enfin du temps passé à la réalisation de cet examen.

Afin de limiter les éléments subjectifs de la méthode manuelle, différents dispositifs automatiques ont été imaginés.

L'un d'entre eux émet successivement des sons à des fréquences fixes. Pour chacune des fréquences, le niveau sonore augmente progressivement. Dès que le sujet signifie qu'il entend le son, c'est-à-dire que son seuil de perception auditive a été dépassé, le niveau du son diminue jusqu'à ce qu'il signifie qu'il ne l'entend plus. Après un temps, le même processus est engagé pour une autre fréquence.

Pour chaque fréquence, les excursions sont enregistrées, représentées sur un graphique qui est ensuite interprété.

Cette technique peut donner de bons résultats lorsque le sujet est particulièrement adroit et attentif, mais l'expérience montre que les excursions réalisées sont souvent de grande amplitude et rendent les audiogrammes très difficilement interprétables.

Les audiomètres nécessitent un étalonnage. Dans certains d'entre eux les couples niveaux/fréquences sont contrôlés à l'aide d'un microprocesseur de telle sorte que la réponse de l'appareil lors d'un balayage en fréquence fournisse une réponse plate (US-A-4 615 007).

Dans un autre type d'appareil automatique mettant en oeuvre la méthode dite "méthode de Bekezy", des sons sont émis à des niveaux croissants et décroissants en fonction de la réponse du sujet examiné. Mais ici, simultanément à la variation du niveau sonore, l'appareil fait glisser la fréquence de façon à explorer un large spectre (par exemple de 125 à 8 000 Hz). Le document US-A-4 038 496 décrit un audiomètre portable permettant la mise en oeuvre de cette méthode.

Cette méthode qui a de nombreux avantages présente également un certain nombre d'inconvénients qui ont empêché sa généralisation. En effet, lors des dépassements du seuil de perception, un son supraliminaire est appliqué ce qui induit un phénomène de saturation comparable à l'éblouissement qui nuit à la précision des mesures suivantes. Par ailleurs, le fait que la balayage en fréquence se poursuive alors que le sujet n'entend pas et que le niveau augmente, entraîne une incertitude de mesure qui peut être importante.

Le but de la présente invention est la réalisation d'un appareil destiné à la mesure de la sensibilité de perception d'un sujet à une vibration, qui permette la réalisation de mesures automatiques ayant une bonne précision et qui évite les inconvénients des appareils précédemment connus.

A cet effet, elle concerne un appareil comportant un émetteur de vibration, un moyen de signalisation actionnable par le sujet lors du franchissement du seuil de perception, une unité de traitement électronique mémorisant la fréquence et le niveau de la vibration lors de chaque franchissement du seuil de perception du sujet et commandant la fréquence et le niveau de la vibration émise.

Selon l'invention, l'unité de traitement explore tout d'abord un niveau de vibration de référence par un balayage en fréquence, il recherche ensuite les seuils de perception correspondant à des niveaux de vibration différents du niveau de référence, par une série de balayages itératifs en fréquence, réalisés en partant de la zone de non perception et prenant fin vers les hautes fréquences et vers les basses fréquences dès que le seuil de perception est atteint.

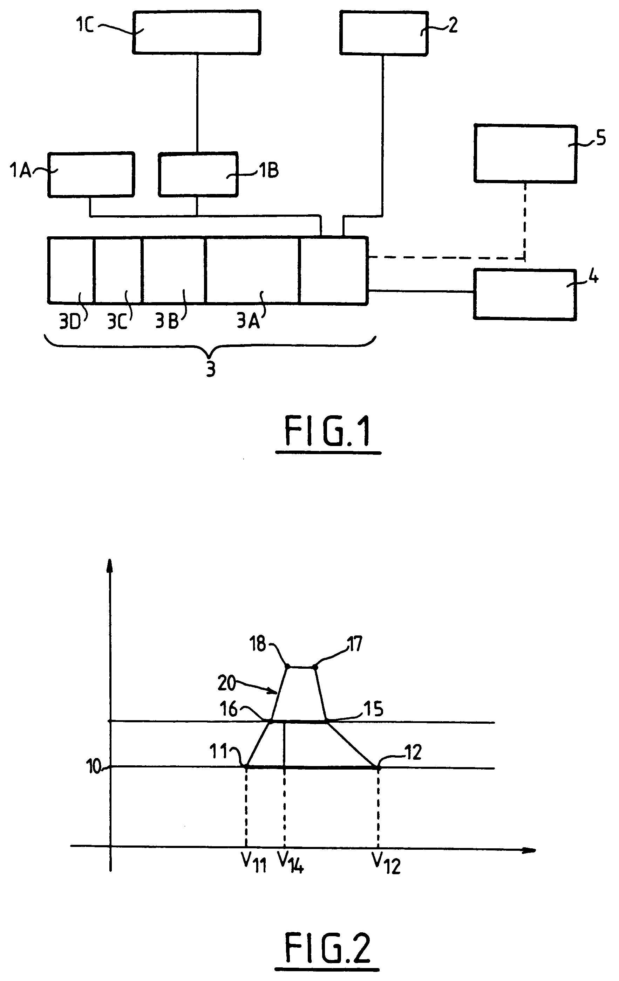

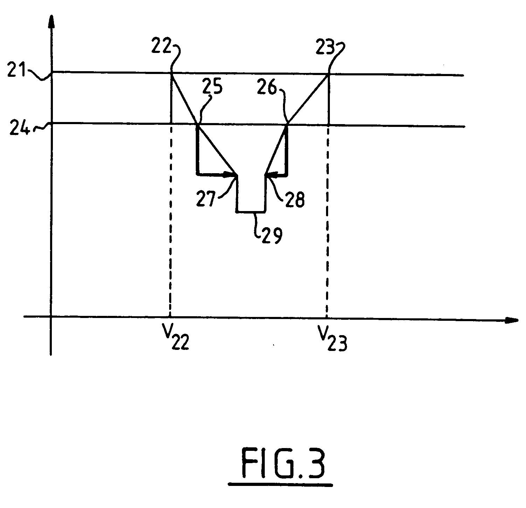

L'invention sera décrite en détail en référence aux dessins annexés dans lesquels :
. La figure 1 est une représentation schématique des éléments de l'appareil de l'invention.
. La figure 2 est une représentation sur un graphique niveau-fréquence des seuils de perception d'un sujet présentant une zone de non sensibilité.
. La figure 3 est une représentation sur un graphique niveau-fréquence des seuils de perception d'un sujet présentant une zone de résonnance.

La description qui suit concerne plus particulièrement un audiomètre. C'est le domaine d'application privilégié de l'invention. Toutefois, elle peut recevoir de nombreuses autres applications, en particulier elle concerne également la mesure des seuils de sensibilité périphérique d'un sujet aux vibrations qui n'est pas un examen encore très répandu mais peut permettre de mieux connaître les mécanismes mis en jeu, de caractériser certains troubles et de participer à l'élaboration de leur réparation.

L'appareil de l'invention est particulièrement bien adapté au dépistage préventif, fournit des résultats reproductibles, indépendants de l'examinateur et permet la réalisation d'examens rapides.

Cet appareil comporte un émetteur de vibration 1 et une presselle 2 utilisée par le sujet comme moyen de signalisation lui permettant de signaler que son seuil de perception a été dépassé. L'émetteur de vibration est de préférence un casque auditif tel que défini dans la norme 350 389 175.

Il comporte une unité de traitement électronique 3. Il s'agit d'un micro-contrôleur par exemple du type commercialisé sur la dénomination "Motorola 68 HC 11" qui comporte une première zone mémoire 3A mémorisant la fréquence et le niveau de la vibration lors de chaque franchissement du seuil d'audition du sujet. L'unité de traitement 3 comporte également une deuxième mémoire 3B destinée à recevoir des valeurs de référence du seuil de vibration correspondant par exemple à une population "normale". Ainsi, il est possible de comparer les seuils d'audition d'un sujet aux valeurs de référence. L'unité de traitement 3 comporte aussi une zone mémoire 3C contenant les données des différents programmes permettant le fonctionnement de l'appareil selon des cycles prédéterminés. Une zone mémoire 3D de l'unité de traitement 3 contient des valeurs d'étalonnage.

Le générateur 1A est un générateur hybride susceptible d'engendrer des sons dont la fréquence est variable de 125 à 16 000 Hz. Il comporte un microprocesseur qui envoie un code de 12 bits sur un convertisseur/numérique analogique. Ce convertisseur numérique/analogique commande un convertisseur tension/fréquence qui génère un signal sinusoïdal. De préférence, la fréquence du signal est asservie de manière à corriger les dérives éventuelles.

L'atténuateur 1B reçoit de l'unité de traitement 3 une valeur d'atténuation élaborée à partir d'une courbe d'étalonnage et de la valeur nominale d'atténuation.

L'unité de traitement 3 commande également la fréquence et le niveau de la vibration émise par l'émetteur 1. Le générateur 1A et l'atténuateur 1B déterminent respectivement la fréquence et le niveau du son émis par le transducteur 1C. L'unité de traitement 3 est reliée à un dispositif de visualisation 4 permettant la représentation de l'audiogramme c'est-à-dire du niveau sonore en fonction de la fréquence correspondant au seuil d'audition du sujet. Ce dispositif de visualisation peut être un écran, une imprimante ou une table traçante. Dans une réalisation préférée, l'appareil comporte un écran à cristaux liquides de 240 points/ligne et 128 lignes qui permet à la fois le contrôle de l'ensemble du fonctionnement de l'appareil et la visualisation, en temps réel, de l'audiogramme du sujet. Il comporte également une imprimante thermique graphique (silencieuse) par exemple de 320 points/ligne.

On entend ici par seuil de perception, le couple de valeurs de paramètres fréquence-niveau de la vibration correspondant pour une fréquence donnée au niveau minimum pour lequel le sujet perçoit la vibration.

Au début de la mesure, l'appareil explore le niveau de référence d'origine 10 par un balayage en fréquence.

Dans le cas, où le sujet, par l'intermédiaire de la presselle 2, signifie qu'il perçoit le signal sur l'ensemble du spectre, l'unité de traitement 3 peut mettre fin à la mesure. Dans le cas d'un dépistage préventif, il peut être alors conclu que le sujet est sain. L'unité de traitement peut aussi poursuivre la mesure en diminuant progressivement le niveau de référence jusqu'à la détection de seuils de perception.

A l'opposé, si le sujet ne montre aucune sensibilité à la vibration au cours de ce balayage en fréquence, l'unité de traitement explore des niveaux de vibrations supérieur par l'émission de sons à quelques fréquences déterminées jusqu'à l'obtention d'une réponse du sujet qui fixe alors le niveau de référence.

Le choix du niveau de référence 10 d'origine et les éventuelles modifications successives de ce niveau de référence sont programmées en fonction du type de mesure que l'on souhaite réaliser.

Lorsque l'unité de traitement reçoit les signaux émis par le sujet lors des franchissements du seuil de perception, il les mémorise et effectue une série de balayages itératifs en fréquence réalisée pour chaque niveau en partant de la zone de non perception et finissant vers les hautes fréquences comme vers les basses fréquences dès que le seuil de perception est atteint.

Ainsi, à la suite de la détection de deux seuils de perception 11 et 12 au niveau de référence 10, séparés par une zone de non-sensibilité, l'unité de traitement commande l'émission de nouvelles vibrations à un niveau 13 supérieur au niveau 10 partant de la fréquence V14 intermédiaire entre les fréquences V12 et V11, c'est-à-dire situé dans la zone de non sensibilité du sujet. Ce balayage vers les hautes fréquences permet la détection du seuil de perception 15 et vers les basses fréquences du seuil de perception 16.

De manière itérative, le processus élaboré à partir des seuils de perception 11 et 12 est reproduit à partir des seuils de perception 15 et 16 permettant la détection des seuils de perception 17, 18...

Les couples de valeurs niveau de vibration-fréquence correspondant aux seuils de perception successivement détectés 11, 12, 15, 16, 17, 18... sont mémorisés et utilisés pour tracer la courbe de sensibilité de perception 20.

Ainsi, l'appareil a réalisé une exploration du "trou" correspondant à la zone de non perception en scrutant alternativement chacune de ses parois. La distance des différents niveaux de référence définit la précision de la mesure finalement réalisée.

Lorsque le sujet présente plusieurs zones de non perception, chacune d'elles sera successivement scrutée.

Un des avantages importants de l'appareil de l'invention qui permet d'obtenir une mesure de bonne qualité est qu'en explorant la sensibilité du sujet à partir de la zone de non perception, il évite tous les défauts susceptibles d'être engendrés par la saturation de la sensibilité du sujet. Simultanément, il présente les avantages résultant des balayages en fréquence. Il permet la réalisation de mesures rapides , soit par un balayage unique permettant de reconnaître les sujets sains (dépistage), soit par une exploration des seules zones porteuses d'informations.

On a jusqu'à présent décrit l'exploration de zones de non perception. Il existe des sujets réagissant au contraire de manière excessive et parfois douloureuse à des vibrations de certaines fréquences. De manière analogue, ces zones de "sursensibilité" peuvent être explorées. Le niveau de référence au lieu d'être progressivement augmenté est alors progressivement diminué.

Ici également, l'appareil de l'invention permet d'éviter les saturations de sensibilité par exemple auditives, l'unité de traitement explore donc les zones de sursensibilité en arrêtant l'émission des vibrations, lorsqu'une telle zone est atteinte.

Lorsque par un balayage à un niveau 21 de référence une zone de résonnance entre deux seuils de perception 22 et 23 est détectée, un nouveau balayage est réalisé par une émission à un niveau inférieur 24. Le balayage est réalisé vers les hautes fréquences à partir de la fréquence V22 (à 5 % près par exemple) du seuil de perception 22 (niveau 21 - basse fréquence) et arrêté dès que le seuil de perception 25 du niveau 24 est atteint. Le balayage vers les basses fréquences est réalisé à partir de la fréquence V23 (à 5 % près par exemple) du seuil de perception 23 (niveau 21 - haute fréquence) et également arrêté dès que le seuil de perception 26 du niveau 24 est atteint. De manière itérative des niveaux de plus en plus faibles sont explorés tant que des seuils de perception sont détectées. Les seuils de perception 22, 23, 25, 26, 27, 28, 29, ... sont mémorisés dans la zone 3A de l'unité de traitement 3.

Un récepteur étalon 5 est utilisé pour l'étalonnage de la chaîne d'émission (unité de traitement 3 - générateur de son 1A - atténuateur 1B - transducteur 1C). Dans le cas des audiomètres, cet étalonnage est normalisé. Les valeurs de cet étalonnage sont conservées dans la zone mémoire 3D. Chaque audiomètre, y compris son casque, fournit une courbe d'étalonnage particulière. Lorsqu'il est utile de pouvoir utiliser un même appareil avec différents casques, différentes zones mémoire 3D, 3D', 3D''... peuvent être prévues, chacune conservant, pour un même appareil la courbe d'étalonnage correspondant à un casque particulier. Avant la mesure, l'utilisateur fournit à l'unité de traitement l'information nécessaire concernant le casque utilisé. La courbe d'étalonnage correspondante est exploitée.

A la fin de l'exploration, la courbe représentant les seuils d'audition sur un graphique niveau-fréquence est visualisée. De préférence, la courbe de référence est également visualisée sur le même graphique, faisant nettement ressortir les écarts de la courbe de sujet avec la courbe de référence. L'appareil de l'invention peut être utilisé pour calculer différents paramètres représentatifs de l'audiogramme, assurer la gestion de ces données en les associant à l'identité du sujet ou encore communiquer avec un système externe par exemple par l'intermédiaire d'une liaison normalisée comme sous l'appellation "RS 332". La communication homme/machine est alors avantageusement réalisée par un clavier et un ensemble de menus déroulants.

## Revendications

1. Appareil destiné à la mesure de la sensibilité d'un sujet à la perception d'une vibration, comportant :
. un émetteur de vibration (1),
. un moyen (2) de signalisation actionnable par le sujet lors du franchissement du seuil de perception
. une unité (3) de traitement électronique, mémorisant la fréquence et le niveau de la vibration de chaque franchissement du seuil de perception du sujet et commandant la fréquence et le niveau de la vibration émise,
caractérisé en ce que
. l'unité de traitement (3) explore un niveau de vibration de référence par un balayage en fréquence,
. puis recherche les seuils de perception correspondant à des niveaux de vibration différents du niveau de référence, par une série de balayages itératifs en fréquence réalisés en partant de la zone de non perception et prenant fin vers les hautes fréquences et vers les basses fréquences dès que le seuil de perception est atteint.

2. Appareil selon la revendication 1, caractérisé en ce que la recherche des seuils de perception est faite pour un niveau donné à partir de la fréquence correspondant à la moyenne géométrique des fréquences des seuils de perception du niveau inférieur précédemment exploré.

3. Appareil selon la revendication 1, caractérisé en ce que la recherche des seuils de perception est faite pour un niveau donné à partir des fréquences des seuils de perception du niveau supérieur précédemment exploré.

4. Appareil selon l'une quelconque des revendications 1 à 3, caractérisé en ce que les différents niveaux explorés sont également répartis.

5. Appareil selon l'une quelconque des revendications 1 à 4, caractérisé en ce qu'il est destiné à la mesure de la sensibilité périphérique d'un sujet, l'émetteur de vibration étant un vibreur électrique.

6. Appareil selon l'une quelconque des revendications 1 à 4, caractérisé en ce qu'il est destiné à la mesure de l'acuité auditive d'un sujet, l'émetteur de vibration (1) étant un émetteur sonore.

7. Appareil selon l'une quelconque des revendications 1 à 5, caractérisé en ce qu'il comporte des moyens (4) de visualisation des seuils de perception mémorisés par l'unité de traitement électronique.

8. Appareil selon la revendication 7, caractérisé en ce que les moyens de visualisation produisant la courbe des niveaux en fonction de la fréquence du seuil de perception.

9. Appareil selon la revendication 8, caractérisé en ce que des valeurs de référence sont visualisées simultanément à la visualisation de ladite courbe.

10. Appareil selon l'une quelconque des revendications 1 à 9, caractérisé en ce que le balayage en fréquence est limité entre une fréquence inférieure et une fréquence supérieure choisies par l'expérimentateur.

## Claims

1. A device intended for measuring the sensitivity of a subject's perception of a vibration, comprising:
a vibration emitter (1),
a signalling means (2) activated by the subject when the perception threshold is crossed
an electronic processing unit (3) which stores the frequency and level of the vibration for each crossing of the subject's perception threshold and which controls the frequency and level of the vibration emitted,
characterised in that
the processing unit (3) explores a reference vibration level through a frequency sweep,
and then searches for the perception thresholds which correspond to vibration levels different from the reference level through a series of iterative frequency sweeps performed starting from the non-perception area and ending around the high and low frequencies as soon as the perception threshold has been reached.

2. A device according to claim 1, characterised in that the search for the perception thresholds is performed for a given level starting from the frequency corresponding to the geometric average of the frequencies of the perception thresholds for the previously explored lower level.

3. A device according to claim 1, characterised in that the search for the perception thresholds is performed for a given level starting from the frequencies of the perception thresholds for the previously explored upper level.

4. A device according to any of claims 1 to 3, characterised in that the various levels explored are equally distributed.

5. A device according to any of claims 1 to 4, characterised in that it is intended for measuring a subject's peripheral sensitivity, with the vibration emitter being an electric buzzer.

6. A device according to any of claims 1 to 4, characterised in that it is intended for measuring a subject's auditory acuity, with the vibration emitter (1) being a sound emitter.

7. A device according to any of claims 1 to 5, characterised in that it comprises means (4) for displaying the perception thresholds stored by the electronic processing unit.

8. A device according to claim 7, characterised in that the display means generate the level curve according to the perception threshold's frequency.

9. A device according to claim 8, characterised in that reference values are displayed simultaneously to the display of said curve.

10. A device according to any of claims 1 to 9, characterised in that the frequency sweep is limited between a lower frequency and an upper frequency selected by the experimenter.

## Patentansprüche

1. Gerät zum Messen der Empfindlichkeit einer Person gegenüber der Wahrnehmung einer Schwingung, mit
- einem Schwingungssender (1),
- einer Meldevorrichtung (2), die von der Person beim Überschreiten der Wahrnehmungsschwelle betätigt werden kann,
- einer elektronischen Verarbeitungseinheit (3), die die Frequenz und den Pegel der Schwingung bei jedem Überschreiten der Wahrnehmungsschwelle der Person speichert und die Frequenz und den Pegel der gesendeten Schwingung steuert,
dadurch gekennzeichnet,
daß die Verarbeitungseinheit (3) durch Frequenzabtastung einen Bezugsschwingungspegel prüft,
und dann die Wahrnehmungsschwellen sucht, welche Schwingungspegeln entsprechen, die vom Bezugsschwingungspegel abweichen, und zwar durch eine Reihe von wiederholenden Frequenzabtastungen, die ausgehend vom Bereich der Nichtwahrnehmung bis hin zu den Hoch- und Niederfrequenzen durchgeführt werden, sobald die Wahrnehmungsschwelle erreicht ist.

2. Gerät nach Anspruch 1, dadurch gekennzeichnet, daß die Wahrnehmungsschwellen für einen gegebenen Pegel gesucht werden, welcher durch die Frequenz bestimmt ist, die dem geometrischen Mittel der Wahrnehmungsschwellenfrequenzen des vorher geprüften unteren Pegels entspricht.

3. Gerät nach Anspruch 1, dadurch gekennzeichnet, daß die Wahrnehmungsschwellen für einen gegebenen Pegel gesucht werden, welcher durch die Wahrnehmungsschwellenfrequenzen des vorher geprüften oberen Pegels bestimmt ist.

4. Gerät nach einem der Ansprüche 1 bis 3, dadurch gekennzeichnet, daß die verschiedenen geprüften Pegel gleich verteilt sind.

5. Gerät nach einem der Ansprüche 1 bis 4, dadurch gekennzeichnet, daß es die Peripherieempfindlichkeit einer Person messen soll, wobei der Schwingungssender ein elektrischer Schwingungserzeuger ist.

6. Gerät nach einem der Ansprüche 1 bis 5, dadurch gekennzeichnet, daß es die Hörschärfe einer Person messen soll, wobei der Schwingungssender (1) ein Tonsender ist.

7. Gerät nach einem der Ansprüche 1 bis 5, dadurch gekennzeichnet, daß es Mittel (4) zur Anzeige der von der elektronischen Verarbeitungseinheit gespeicherten Wahrnehmungsschwellen aufweist.

8. Gerät nach Anspruch 7, dadurch gekennzeichnet, daß die Anzeigemittel die Pegelkurve in Abhängigkeit von der Frequenz der Wahrnehmungsschwelle erzeugen.

9. Gerät nach Anspruch 8, dadurch gekennzeichnet, daß die Bezugsgrößen zum gleichen Zeitpunkt wie die besagte Kurve angezeigt werden.

10. Gerät nach einem der Ansprüche 1 bis 9, dadurch gekennzeichnet, daß die Frequenzabtastung auf einen Bereich zwischen einer unteren und einer oberen Frequenz beschränkt ist, die vom Experimentator gewählt werden.
